# EUROPEAN PATENT APPLICATION

(11) **EP 0 774 233 A1**
(43) Date of publication of application: **21.05.1997**
(21) Application number: 95308191.6
(22) Date of filing: 15.11.1995
(51) Int. Cl.: A61B 5/08, A61B 5/083

(54) **Method of preventing the formation of a dangerous underpressure in a respiratory system**

(71) Applicant: INSTRUMENTARIUM CORPORATION, SF-00510 Helsinki (FI)
(72) Inventor: Hakala, Matti, c/o Instrumentarium Corp., FIN-00510 Helsinki (FI)
(74) Representative: Charlton, Peter John

(57) **Abstract**

A method of preventing the formation of dangerous underpressure in a respiratory system, which comprises a respirator (1) connected to the patient, and a sampling device (11) and a gas analyzer (3) connected thereto, in which method inspired and expired air is aspirated at a predetermined pressure for sampling through the sampling line (7). When an excess underpressure is formed in the gas analyzing unit, the aspiration of the pump (2) is prevented in the respiratory tube (15).

## Description

The objective of the invention is a method of preventing the formation of dangerous underpressure in a respiratory system.

When the patient is connected to a respirator, a pressure generally prevails which is at least the same as the ambient pressure. During inspiration an overpressure is required to fill the patient's lungs, and the pressure decrease is often even restricted in the expiration phase in order to prevent the collapsing of the patient's lungs, for which is used the term PEEP, Positive End-Expiratory Pressure. The occurrence of underpressure, on the other hand, is exceptional in such a system and is generally considered as injurious. At the worst underpressure can cause absorption of fluid from the tissue to the lungs and lead to permanent injuries. The allowed underpressure is only some water centimeters.

In connection with respiratory breathing it is nowadays customary to measure the concentrations of gases inspired and expired, especially the carbon dioxide, oxygen and anesthetic gas concentrations. This is generally made by aspiring a small amount of gas, typically about 200 ml/min into a monitor that analyzes the concentrations of the desired gases and displays the result on e.g. a display.

The amount of sample gas taken by the gas monitor is in normal conditions insignificant compared to the respiratory gas amount fed into the system, the so called fresh gas flow, which generally is several liters per minute. Even in extreme cases, when the fresh gas flow is below 1 l/min., when a so called low flow anesthesia is in question, the gas amount discharged from the system can easily be substituted by increasing the fresh gas flow correspondingly.

There are, however, cases when due to human error or equipment failure the gas feed into the system has been interrupted. The gas amount aspired by the monitor is in such cases not substituted at all, but the monitor aspirates into the respiratory system a growing underpressure, which is limited only by the capability of the pump to generate underpressure, and on the other hand by a possible leakage in the system, which again in itself is not a desired property. The underpressure can, dependent on the gas volume of the system, fairly quickly reach a limit where the patient's safety for the above reasons is jeopardized.

There has been no previous method available to secure the prevention of such an underpressure formation. The gas monitors generally measure the pressure of their own internal sampling system, which in principle is also dependent on the pressure of the patient system. The dependence is, however, not unambiguous, as the difference between these pressures also depends e.g. on the flow resistance in the sampling line. This sudden pressure decrease is generally caused by constriction in the sampling line, wherefore it cannot reliably be used to indicate the pressure prevailing in the patient tube system.

New respirators have usually a built-in airway pressure monitoring, some also provided to alarm when the pressure falls below a preset limit. There are, however, quite many old devices in use which lack these properties. Moreover, it is quite possible that despite monitoring and alarm, the locating of the problem and the repair of the failure take so long that the patient will already suffer from the situation. The ventilator patient tube might also constrict, thus putting the ventilator safety equipment out of function. No means have been provided so far to prevent the occurrence of such a situation.

The method according to the invention provides a decisive improvement of the above mentioned disadvantages. The invention is characterized in what is presented in the claims above.

The invention provides a considerable improvement of the safety of a patient connected to a respirator. The invention provides preferably separately an error message about a constriction in the sampling line as well as of underpressure in the respiratory tube. The patient circuit underpressure can be identified by the invention to differ from a constriction in the sampling line. The underpressure error message can quickly be located with the subject invention.

The invention is below presented with reference to the enclosed drawing, in which
- fig. 1: presents a basic view of a respirator and a gas measuring device,
- fig. 2: presents an alternative of the solution of fig. 1,
- fig. 3A and B: show different connections of the sampling line to the respiratory tube or to the adapter,
- fig. 4: presents gas measuring options to be connected to the respiratory tube, and
- fig. 5: shows a more detailed view of the underpressure indicating device of fig. 4.

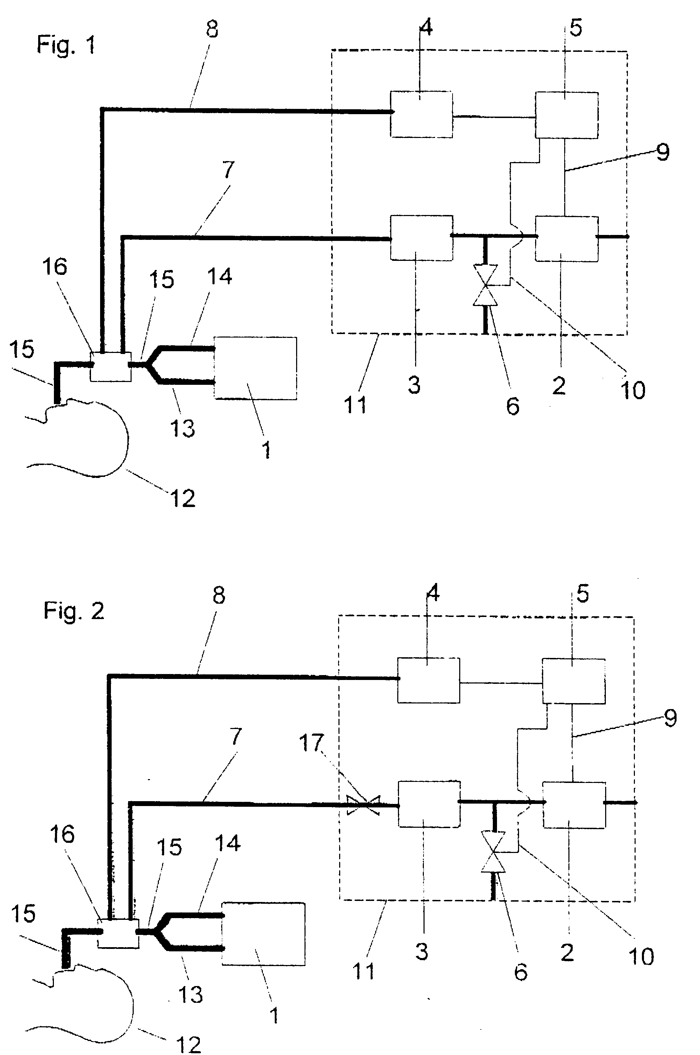

Fig. 1 presents one favorable embodiment of the invention, in which a patient 12 is connected to a respirator 1. The respirator comprises an inspiration tube 13, which carries inspiration air or oxygen to the patient, and an expiration tube 14, which returns the expiration gases from the patient back to the respirator. The tubes 13, 14 are close to the patient connected into one tube 15, which as such or through an adapter attached thereto can be connected to an intubation tube, which through the patient's mouth or nose can be led to the trachea or to the mask. To this combined tube 15, or to the adapter 16 attached thereto, has then been connected a sampling line 7, through which are aspirated samples of the patient's respiratory gas, which can be the inspiration and/or the expiration gas. The cross-sectional area of the opening provided for the sampling in the adapter 16 or in the tube 15, or of the sampling line 7 extending from the opening is qenerally smaller than the cross-sectional area of the tube 15. The sample is preferably aspirated through the sampling line by the pump 2 of the monitor 11 to the gas analyzing unit 3, in which the patient's inspired and/or expired gas is analyzed. The analyzing can comprise the measuring or identifying, or both, of one or several gas component concentrations. Carbon dioxide, oxygen and anesthetic gases are generally analyzed.

A second line 8 for measuring the pressure is preferably connected to the tube 15 or to the adapter 16 attached thereto. A member 4 observing the underpressure and generally called the pressure sensor, is attached to the pressure measuring line 8 to measure the pressure caused by the gas in the respiratory tube system. The pressure measuring can be carried out by comparing the pressure prevailing in the respiratory tube 15 with the pressure outside the tube, i.e. the ambient pressure. The gas sampling pump 2 is controlled through the signal way 9 from the control unit 5, which receives information about the pressure variation. According to the invention, when the information about the gas pressure is below a certain predetermined pressure, the sampling from the respiratory tube 15 connected to the patient is prevented. This can be realized e.g. by stopping the pump. The control unit can simultaneously send a signal through the connection 10 to the valve 6, through which there is a connection to the ambient air, to open the valve to prevent the underpressure possibly formed in the tubes from injuring the patient. The pressure of the lines 7 and 8 is then the same as the ambient pressure, and the disadvantages caused by the aspiration can be eliminated.

The pressure measuring could as well take place locally in the tube 15 or in the adapter 16, whereby the line 8 would not be needed. The tube could then be replaced by a wire to transfer the signal to the control unit 5.

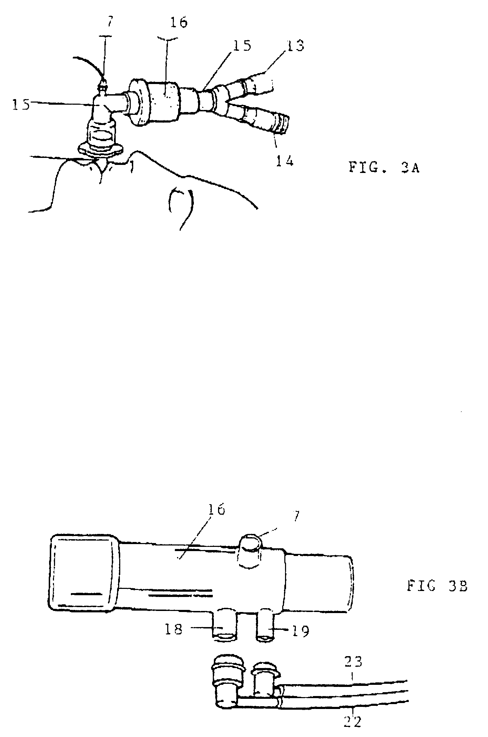

There are also other means available than the ones presented above to prevent the formation of underpressure injurious to the patient. The pump could remain in function, but when the gas pressure falls below the predetermined value, the aspiration from the respiratory tube 15 would be prevented by a valve 17 provided between the pump and the respiratory tube. This valve could simply be an open-closed valve according to fig. 2. The flow connection between the patient and the pump is then closed. It would further be sufficient to prevent the formation of underpressure by turning the valve 6 into open position to enable the ambient gas to flow through this valve 6 into the line 7. A sufficient flow through the valve 6 would make the stopping of the pump almost unnecessary. Another alternative solution could be based on the valve 17 located in the line 7 between the pump and the respiratory tube, which valve when aspirating the sample could be in a position where the pump aspiration would be connected to the respiratory tube, but due to the formed dangerous underpressure in the respiratory tube, the valve 17 would be connected to aspirate the ambient gas, thus interrupting the aspiration from the respiratory tube.

A favorable device for the embodiment of the method according to the invention has been presented above, in which device an airway pressure measuring by means of a pressure sensor 25 has been connected to a gas monitor. Due to this the device has constantly current information about the pressure prevailing in the respiratory tube system and thereby also in the airway. The gas sampling pump can be controlled with the information provided by the pressure sensor. The valve 6, in one favorable embodiment of the invention, can be used when desired, as often is the case, to aspirate room air to the device instead of sampling gas from the patient for the calibration of the measuring.

The concentrations of carbon dioxide, oxygen, nitrous oxide and anesthetic agents can, when necessary, be measured or identified in the gas measuring unit. The measuring units are in connection with a microprocessor enabling the generating of the alarms and/or the collecting of data. Before the gas reaches the measuring unit, water is separated from the gas into a water trap, the construction and function of which have been described in e.g. the US-patents 4304578, 4382806 and 4886528. In the water trap, the sample flow can be divided into two parts, a flow to be analyzed and a side-flow. The flow quantity to the analyzing devices is usually controlled by a throttle. The gas pressure and flow measuring analytics, when a separate pressure measuring line is in question, is also in connection with the control unit microprocessor, from which there is, according to the invention, a connection to the pump to control the pump so that underpressure is not generated in the respiratory tubes because of the pump. A key-board is normally attached to the processor for the feeding of the necessary information, as well as a display and necessary loudspeakers for the alarms to inform about e.g. values above and below defined limit-values.

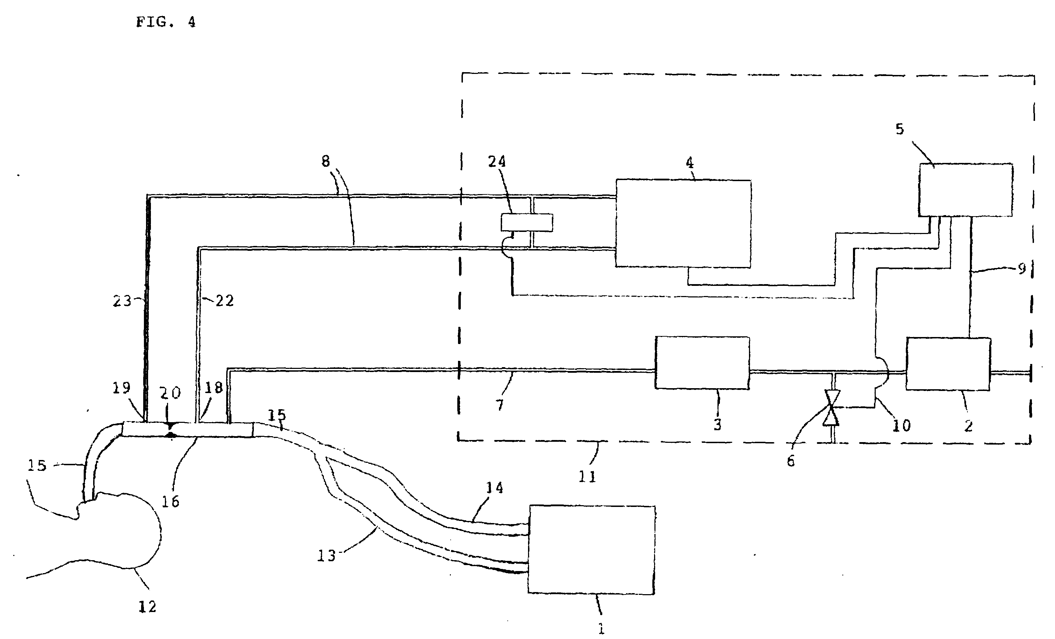

Figs. 3 A and B present different possibilities of connecting the sampling lines to the respiratory tubes, when either one or several tubes are used. Fig. 3 A shows a respiratory tube 15 connected to the patient and the respiratory tubes 13 and 14 with an adapter 16 in between. A sampling line 7 is provided in the adapter 16 opening. Fig. 3 A shows a typical endotracheal intubation. A trachsostomy tube or a mask ventilation system can also be used as well as paediatric respiratory systems.

Fig. 3 B presents one favorable adapter alternative 16 to be connected to the respiratory tube 15. This is a flow sensor, the construction and function of which have more specifically been described in the US-patents 5111827, depicted in fig. 4, and 5088332. The flow measuring thus takes place by measuring the pressure difference over the throttle 20. For this purpose, the side of the adapter is provided with two openings 18, 19, through which the pressure signal received from the different sides of the throttle 20 is led through the line 8 to the pressure difference observing member 21. The line 8 has then two channels 22, 23. Either one of these channels can further be connected to the pressure sensor 25, which thus compares the pressure in the respiratory tube with the ambient pressure. A connection is provided between the channels 22, 23, which can be controlled by a magnet valve 24. The member 21 observing the pressure difference prevailing in the tube 15 over the throttle 20 can also be used instead of the sensor 25 to observe the underpressure in the respiratory tube, because the pressure difference over the throttle deviates considerably from normal condition due to the dangerous underpressure generated in the respiratory tube. The signal received from the pressure difference observing member 21 can be transmitted to the control unit, because the pressure difference observing member 21 is connected with a wire to the control unit 5. A sampling line 7 is connected to the third opening in the adapter for the aspiration of the sample to the analyzing unit. An appropriate technique is naturally required to generate the signal and to transmit it to the control unit. The adapter shown in fig. 4 can be used to measure the pressure either by measuring the flow magnitude or the pressure difference, or both can be used together. The flow measuring can e.g. compare the behavior of the flow as a function of time.

The adapter can e.g. be used for common endotracheal intubation. The adapter in fig. 3 B is known by the Instrumentarium Oy's brand name D-LITE™ or PEDE-LITE™.

A dangerous underpressure is generally in the magnitude of - 20 cmH₂O, but already e.g. an underpressure value of -5 cmH₂O can cause emergencies, if the effect of the underpressure is for more than one minute. The ambient pressure of the tubes is generally the 0-level, which is the atmospheric pressure, i.e. approx. 1 atm. The expiration pressure is generally the same as the ambient pressure and at inspiration smaller than 30 cmH₂O.

The flow can alternatively be measured so that, knowing that the expiration and inspiration flows follow predetermined curves, a sudden change in the flow depicting curve in relation to comparative curves could cause an alarm of underpressure in the respiratory tube. The underpressure thus generates an error and alarm message.

Different alternatives are possible. The adapter 16 provided in the respiratory tube 15 can be according to fig. 4, with which is measured either the pressure or the flow magnitude or both together. The adapter 16 can be attached from its one end either to the intubation tube or direct to the mask or to endotracheal intubation, which are not separately shown in the pictures. Different connectors might be required or a respiratory tube as in the figs. 1 and 2.

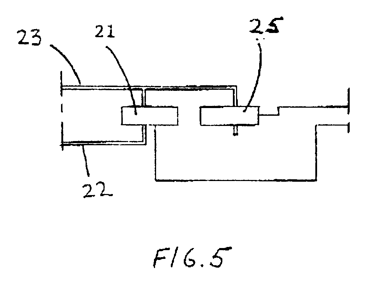

Fig. 5 presents a partial view of fig. 4. The underpressure observing member 4 can be a pressure or flow measuring device, e.g. the device 21, which measures the flow by observing the pressure difference over the throttle 20 provided in the adapter 16, and a sensor 25 comparing the tube pressure with the ambient air. Both can be used together for the measuring or only one of them.

The invention has above been described with reference to only one of its embodiments. The invention is not to be considered as so restricted, but all modifications and alternatives, e.g. in the flow measuring, are possible within the scope of the inventive idea according to the claims below.

## Claims

1. A method of preventing the formation of dangerous underpressure at sampling in the patient's airway when the patient is connected to a respirator (1), in which method samples are taken from the patient's respiratory gas by aspirating the sample through the sampling line (7) to the gas analyzing unit (3),
the magnitude disclosing the desired underpressure prevailing in the airway is measured, characterized in that when the magnitude value of the measurement indicates a value below the predetermined underpressure value in the respiratory tube (13, 14, 15), the sampling from the respiratory tube (13, 14, 15) connected to the patient is prevented.

2. A method according to claim 1, characterized in that either the pressure or the flow in the respiratory tube (13, 14, 15) is measured, which tube is in connection with the patient's airway.

3. A method according to claim 2, characterized in that the pressure is measured by comparing the pressure prevailing in the respiratory tube (15) with the ambient pressure.

4. A method according to claim 3, characterized in that information about the measuring is given to the control unit (5), from where, when the pressure is below the predetermined value, instructions are given to prevent the formation of underpressure in the airway.

5. A method according to claim 2, characterized in that the flow is measured by observing the pressure difference over a throttle (20) provided in the respiratory tube (15).

6. A method according to claim 5, characterized in that through the two openings in the adapter (16), which are located on the different sides of the throttle (20), the pressure signal received from different sides of the throttle (20) is transmitted to the pressure difference observing member (21).

7. A method according to claim 6, characterized in that the information of the pressure difference observing member (21) is transferred to the control unit (5), which, when receiving information about a pressure below the predetermined limit value, prevents the formation of underpressure in the airway.

8. A method according to claim 7, characterized in that the pump (2) is stopped when the pressure falls below a predetermined value to prevent the formation of underpressure in the respiratory tubes (13, 14, 15).

9. A method according to claim 8, characterized in that when the pump (2) stops, the valve (6) leading from the sampling line (7) to the ambient air is opened.

10. A method according to claim 1, characterized in that gas is aspirated through the gas sampling line (7) to the gas analyzer (3), and the airway pressure is measured through a separate other line connection (8) by means of a pressure sensor (25), based on which information, if it is below the predetermined pressure, the pump (2) aspiration through the sampling line (7) is prevented.

11. A method according to claim 10, characterized in that the pressure in the sampling line (7) and in the airways is balanced when the pump stops by opening the valve (6), through which ambient air pressure is coming to the line (7).

12. A method according to claim 1, characterized in that the valve (6) is also used when ambient room air is desired to be aspirated to the device (11) instead of sample gas from the patient for the calibration of the measurements.

13. A method according to claim 1, characterized in that when the pressure value measured from the airway falls below a predetermined value, information about this is transferred to the unit (5) controlling the pump (2), from which unit signal is given so that the running of the pump (2) is prevented or the formation of underpressure is prevented in the gas sampling line (7).

14. A method according to claim 1, characterized in that the formation of an excess underpressure because of the gas sampling pump (2) is prevented by opening the control valve (6) connected to the sampling line (7).

15. A method according to claim 1, characterized in that between the gas analyzing unit (3) and the respiratory tube (15, 16) is provided a valve (17), from which there is alternatively access to the ambient air or it forms a connection between the respiratory tube (15, 16) and the gas analyzing unit (3), so that it is opened when the pressure falls below a predetermined value, whereby the connection between the pump (2) and the tube (15) is prevented.

16. A method of preventing the formation of dangerous underpressure in the patient's airway when the patient is connected to a respirator (1), in which method samples are taken from the patient's respiratory gas by aspirating the sample through the sampling line (7) to the gas analyzing unit (3),
the underpressure prevailing in the airway is measured, characterized in that
when the measurement indicates a value below the predetermined underpressure value in the respiratory tube (13, 14, 15), the sampling from the respiratory tube (13, 14, 15) connected to the patient is prevented.

17. A method of preventing the formation of dangerous underpressure in the patient's airway when the patient is connected to a respirator (1), in which method samples are taken from the patient's respiratory gas by aspirating the sample through the sampling line (7) to the gas analyzing unit (3),
the respiratory gas flow is monitored, characterized in that when the information provided by the flow indicates a value below the predetermined underpressure value in the respiratory tube (13, 14, 15), the sampling from the respiratory tube (13, 14, 15) connected to the patient is prevented.
